# EUROPEAN PATENT APPLICATION

(11) **EP 4 813 444 A1**
(43) Date of publication of application: **30.09.2026**
(21) Application number: 24894634.5
(22) Date of filing: 21.11.2024
(51) Int. Cl.: A61N 1/372, A61N 1/05, A61N 1/36, A61N 1/375, H04B 13/00, H04B 5/72, H04B 5/79

(54) **IMPLANTABLE ELECTROCEUTICAL SYSTEM INCLUDING CORTICAL ELECTRODE AND WIRELESS CHARGING DEVICE**

(30) Priority: 21.11.2023 KR 20230162082
(71) Applicant: Gbrain Inc., Incheon 21984 (KR)
(72) Inventor: YANG, Sunggu, Incheon 22018 (KR); KIM, Byoung Kwan, Incheon 21986 (KR); LEE, Kyu Il, Incheon 21982 (KR); LEE, Sangwon, Incheon 21994 (KR); KIM, Minji, Incheon 22146 (KR); KIM, Kyung Tae, Incheon 21363 (KR)
(74) Representative: Patrade A/S
(86) International application number: PCT/KR2024/018482
(87) International publication number: WO 2025/110742

(57) **Abstract**

The present invention includes an implantable element including a contact portion configured to measure a signal generated in the brain or deliver stimulation to the brain, a transmission and reception portion configured to transmit, to the outside, a signal received from the contact portion or deliver, to the contact portion, a signal indicating the stimulation, and a connection portion connecting the contact portion and the transmission and reception portion; and an integrated circuit module connected to the transmission and reception portion and configured to transmit and receive signals, wherein the integrated circuit module has a stacked structure.

## Description

### Technical Field

The present invention relates to an implantable electroceutical system including a cortical electrode and a wireless charging device. The present invention was researched with support from the following projects.
1. Alchemist Project ([Unique Project Number] 20012355 / [Project Number] 20012355 / [Ministry] Ministry of Trade, Industry and Energy / [Project Management Specialized Agency] Korea Evaluation Institute of Industrial Technology / [Research Program] Industrial Technology Alchemist Project / [Research Project Title] Development of Fully Implantable Closed Loop- Brain-to-X for Voice Communication / [Contribution Rate] 100% / [Research Institution] Gbrain Inc. / [Research Period] September 1, 2020 to December 31, 2026 (total research period))
2. Materials and Components Technology Development Project ([Unique Project Number] RS-2024-00418941 / [Project Number] RS-2024-00418941 / [Ministry] Ministry of Trade, Industry and Energy / [Project Management Specialized Agency] Korea Institute for Industrial Technology Planning and Evaluation / [Research Program] Materials and Components Technology Development (R&D) / [Research Project Title] Wireless Closed-Loop Neurostimulator for Diagnosis and Treatment of Parkinson's Disease / [Contribution Rate] 100% / [Research Institution] Gbrain Inc. / [Research Period] July 1, 2024 to December 31, 2026)

### Background Art

A brain-implantable medical device is a device including multiple microelectrodes for obtaining neural signals or delivering electrical stimulation, and serves as a neural interface that connects neurons to an electronic circuit. Electrical stimulation has advantages of fewer side effects, reversibility, and ease of adjustment compared to drug therapy or ablation.

Currently commercialized brain-implantable medical devices are rigid and bulky, and thus require a large opening of the skull when inserted into the brain and require the use of a cerebral penetrating electrode probe for stimulating a deep brain region. Accordingly, there have been problems in that side effects such as changes in intracranial pressure due to opening of the skull, serious damage to the deep brain region, and infection may occur.

In addition, when a brain-implantable medical device is inserted into the brain, a connection line to an external side causes discomfort to a patient, and there have been inconveniences such as a need to perform a separate connection procedure with the external side.

Therefore, in the art, continuous and active research and development are being conducted to supplement the problems of brain-implantable medical devices.

### Disclosure

### Technical Problem

The present invention has been created to solve the above-described problems of the related art and expand the range of application, and an object of the present invention is to provide a medical device for measuring brain signals and delivering stimulation, which has a minimum volume by using a high-performance element having excellent adhesion to biological tissue and a stacked structure design, thereby minimizing opening of the skull.

### Technical Solution

The present invention includes an implantable element including a contact portion configured to measure a signal generated in the brain or deliver stimulation to the brain, a transmission and reception portion configured to transmit, to the outside, a signal received from the contact portion or deliver, to the contact portion, a signal indicating the stimulation, and a connection portion connecting the contact portion and the transmission and reception portion; and an integrated circuit module connected to the transmission and reception portion and configured to transmit and receive signals. The integrated circuit module has a stacked structure.

Specifically, the integrated circuit module may include a housing and a stacked portion having the stacked structure. The housing may include an upper case configured to protect an upper side of the stacked portion, a side case having a height greater than a height of the stacked portion and including a through portion in at least a portion of one side thereof, and a lower case formed below the stacked portion. The side case may be formed to be larger than the stacked portion such that a space is at least partially formed between the side case and the stacked portion.

Specifically, the transmission and reception portion may be formed such that at least a portion thereof passes through the through portion.

Specifically, the upper case may be formed of a synthetic resin material, and the side case and the lower case may be formed of a metal material.

Specifically, the stacked portion may include a receiver coil, a battery, an IC chip, a printed circuit board (PCB), and at least a portion of the transmission and reception portion.

Specifically, the transmission and reception portion of the stacked portion may be formed at a lowermost layer, and the stacked portion may be stacked in the order of the transmission and reception portion, the PCB, the IC chip, the battery, and the receiver coil.

Specifically, the battery may be formed in a circular shape, and the housing may be provided in a rectangular shape.

Specifically, each component of the stacked portion may be electrically connected.

### Advantageous Effects

In a structure for measuring brain signals and providing stimulation, the structure including the implantable element and the integrated circuit module of the present invention, the implantable element has excellent flexibility and mechanical properties and is seated on the cerebrum. Accordingly, surgery can be performed only by opening a very narrow area of the skull, thereby improving surgical safety, and patient discomfort can be minimized by minimizing a volume of the structure including the integrated circuit module installed in the skull.

### Description of Drawings

FIG. 1 is a view illustrating a configuration of an implantable element according to an embodiment of the present invention.
FIG. 2 is a view illustrating a form in which an integrated circuit module according to an embodiment of the present invention is attached to a surface of a skull.
FIG. 3 is a view schematically illustrating an internal configuration of an integrated circuit module according to an embodiment of the present invention.
FIG. 4 is a view schematically illustrating a system including a cortical electrode and an inductive-coupling-based wireless device according to another embodiment of the present invention.
FIG. 5 is a view schematically illustrating an example of an implantable element according to another embodiment of the present invention.
FIG. 6 is a view schematically illustrating another example of an implantable element according to another embodiment of the present invention.
FIG. 7 is a view schematically illustrating still another example of an implantable element according to another embodiment of the present invention.
FIG. 8 is a view schematically illustrating an example of a left end portion according to another embodiment of the present invention.
FIG. 9 is a view schematically illustrating an example of a connection portion according to another embodiment of the present invention.
FIG. 10 is a view schematically illustrating another example of a connection portion according to another embodiment of the present invention.
FIG. 11 is a view schematically illustrating still another example of a connection portion according to another embodiment of the present invention.
FIG. 12 is a view schematically illustrating an example of a system including a guide portion according to another embodiment of the present invention.
FIG. 13 is a view schematically illustrating a guide portion including a fixing member according to another embodiment of the present invention.
FIG. 14 is a view schematically illustrating another example of a system according to another embodiment of the present invention.
FIG. 15 is a view schematically illustrating another example of a system according to another embodiment of the present invention.
FIG. 16 is a view schematically illustrating a system including a cortical electrode and an inductive-coupling-based wireless device according to still another embodiment of the present invention.

### Best Mode

The objects, specific advantages, and novel features of the present invention will become more apparent from the following detailed description and preferred embodiments taken in conjunction with the accompanying drawings. In assigning reference numerals to components in the respective drawings in the present specification, it should be noted that the same components are assigned the same reference numerals as much as possible even when they are illustrated in different drawings. In addition, in describing the present invention, when a detailed description of related known art is determined to unnecessarily obscure the gist of the present invention, the detailed description thereof will be omitted.

Hereinafter, the detailed description will be provided with reference to the drawings illustrated below.

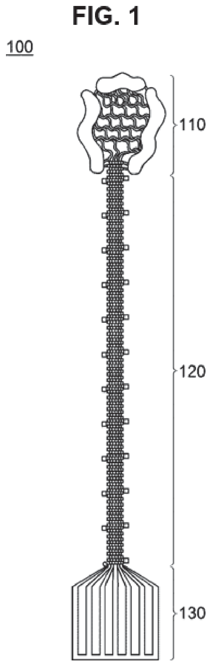

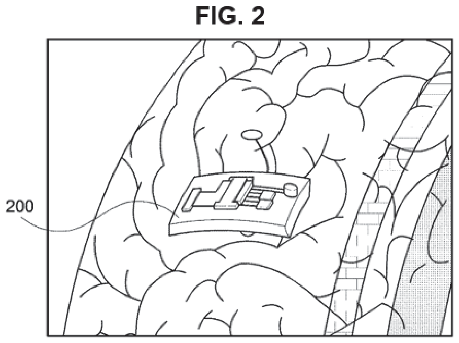

FIG. 1 is a view illustrating a configuration of an implantable element according to an embodiment of the present invention, and FIG. 2 is a view illustrating a form in which an integrated circuit module according to an embodiment of the present invention is attached to a surface of a skull.

A system 10 including a cortical electrode and an inductive-coupling-based wireless device according to an embodiment of the present invention includes an implantable element 100 and an integrated circuit(integrated circuit module) 200. In addition, the system 10 including the cortical electrode and the inductive-coupling-based wireless device may be a structure for measuring brain signals and providing stimulation, or may be a system including the same.

Referring to FIG. 1, the implantable element 100 according to an embodiment of the present invention may include a contact portion 110, a connection portion 120, and a transmission and reception portion 130. Specifically, the implantable element 100 may include the contact portion 110 configured to contact a surface of the cerebral cortex, the transmission and reception portion 130 installed in a space between the skull and skin, and the connection portion 120 connecting the contact portion 110 and the transmission and reception portion 130 at opposite ends thereof and disposed across an inside and an outside of the skull by passing through the skull. Here, the implantable element 100 is a flexible cortical electrode and may be deformable into various forms, and embodiments of the respective forms will be described below.

The system 10 including the cortical electrode and the wireless device according to the present invention may include components for a flexible electrode, a wireless link, wireless power supply, recording, and stimulation.

Among the implantable elements 100 inserted into the brain, the contact portion 110 is brought into contact with the cerebral cortex, and the connection portion 120 passes through the skull and connects the contact portion 110 and the transmission and reception portion 130. The contact portion 110 may include an electrode pad and may contact the cerebral cortex through the electrode pad.

The transmission and reception portion 130 is coupled to the integrated circuit 200, and is positioned between the skull and the skin, on the skull, or to be inserted into the skull.

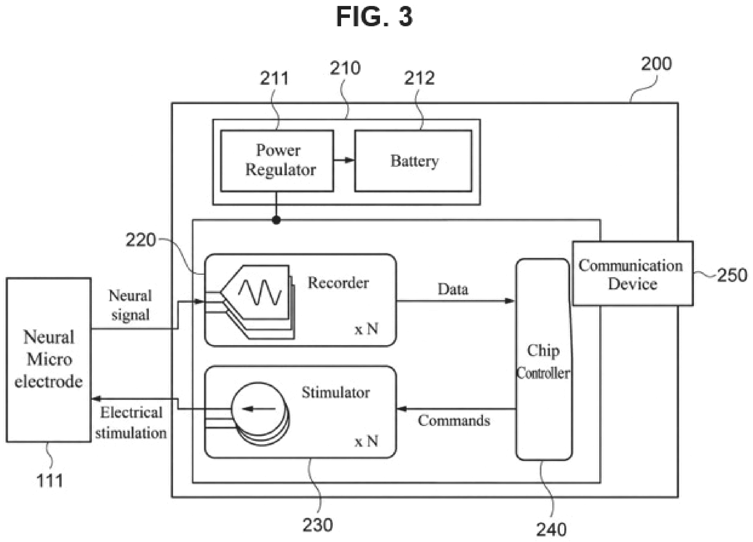

FIG. 3 is a view schematically illustrating an internal configuration of an integrated circuit 200 according to an embodiment of the present invention.

The integrated circuit 200 according to an embodiment of the present invention may be a wireless chip, and serves to wirelessly transmit and receive radio waves or wirelessly receive power. The integrated circuit 200 may include a wireless power device, a recording device, a stimulation device, and a wireless communication device, and may be a package capable of improving biocompatibility by including the above-described components.

The integrated circuit 200 is installed in a space between the skull and the skin. Specifically, the wireless power device is for supplying power to the integrated circuit 200, the recording device is for recording measured brain waves, the stimulation device is for processing a signal for providing stimulation to the brain, and the wireless communication device is a component for wirelessly transmitting the measured brain waves to the outside and receiving an external command.

Referring to FIG. 3, the integrated circuit 200 records measured brain signals, obtains biological information, and detects an abnormal signal. When an abnormality is detected, the integrated circuit 200 applies energy such as current stimulation, voltage stimulation, magnetic-field stimulation, or electric-field stimulation to perform neuromodulation, thereby treating a disease or controlling brain activity.

The integrated circuit 200 may include a wireless chip power supply portion 210, which is a wireless power device, a recorder 220, which is a recording device, a stimulator 230, which is a stimulation device, a chip controller 240, and a communication device 250.

The wireless chip power supply portion 210 includes a power regulator 211 and a battery 212.

The battery 212 may enable wireless energy harvesting by using an RF coil, an on-chip coil, or the like, and may store energy supplied in a wireless manner.

The battery 212 may be wirelessly charged by using wireless power transfer (WPT) technology.

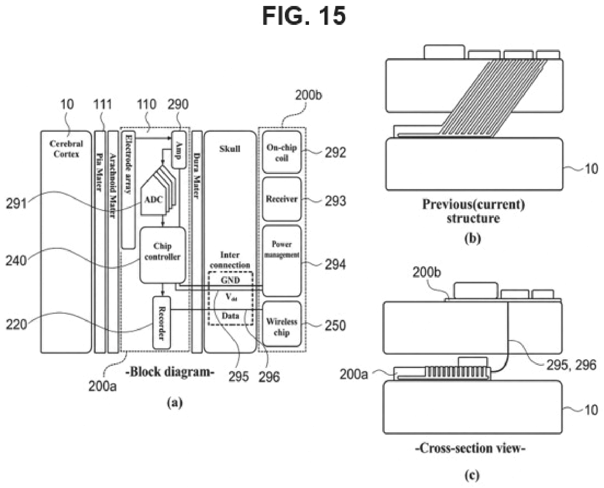

The power regulator 211 is a current converter, and may convert AC of the battery 212 into DC and directly deliver the DC to the chip controller 240, or may deliver the DC to a power management circuit 294 of FIG. 15 and then to the chip controller 240.

A neural microelectrode is an electrode 111 attached to the brain and is capable of measuring brain waves and providing electrical stimulation, and may be, for example, the electrode 111 formed in the contact portion 110 of the implantable element 100.

In brain wave measurement, analog data measured by the neural microelectrode is converted into digital data by an analog-to-digital converter (ADC) 291 of FIG. 15, and is delivered to the chip controller 240. In this case, the digital data may be recorded by the recorder 220, and the recorded digital data may be wirelessly transmitted to external equipment through the wireless communication device 250 via the chip controller 240. Here, the analog-to-digital converter (ADC) 291 may vary depending on the number of brain wave measurement panels, that is, the number of electrodes 111, and may be configured to be included in the recorder 220.

The recorder 220 may perform input of multi-channel brain wave measurement data and digital signal conversion through the analog-to-digital converter (ADC) 291.

The chip controller 240 may control a measurement signal and a stimulation signal.

The wireless communication device 250 is wirelessly connected to an external communication network to enable monitoring outside the body. The wireless communication device 250 may wirelessly transmit the digital signal converted by the analog-to-digital converter (ADC) 291 of the recorder 220 through an electrode or an antenna.

In consideration of problems such as packaging heat generation, the integrated circuit 200 should secure biocompatibility by allowing a package material of the integrated circuit to have biocompatibility with a living body, and should not affect the chip during formation of encapsulation.

The integrated circuit 200 may use, without limitation, any material generally used in vivo in the art as a package material, and polydimethylsiloxane (PDMS), Parylene C, polyimide, biocompatible UV resin, or the like is suitable.

Specifically, when brain waves are measured through the electrode 111 exposed to the outside from the contact portion 110 of the implantable element 100, for example, through a graphene electrode layer, and are delivered to the integrated circuit 200, the brain waves may be wirelessly transmitted to the outside through the integrated circuit 200.

When an abnormality is detected in a brain wave signal measured by the chip controller 240, the brain wave signal is transmitted to the outside through the wireless communication device 250, and a command is delivered to the stimulator 230 to apply an electrical stimulation signal to the neural microelectrode, that is, the electrode 111. The stimulator 230 varies depending on the number of electrical stimulation channels. The stimulator 230 may generate or deliver therapeutic stimulation instructed by the chip controller 240.

Brain waves measured through the contact portion 110 of the implantable element 100 may be converted into digital signals through the recorder 220 and delivered to the integrated circuit 200, and may be wirelessly transmitted to a smart device such as a smartphone or a smart pad, a computer, or the like. One or more recorders 220 may be included.

Conversely, radio waves received from the outside through the integrated circuit 200 may be delivered to the brain through the implantable element 100.

Through this process, when an abnormality is detected in the brain waves measured by the contact portion 110, the abnormality is transmitted to the outside, and the integrated circuit 200 may be controlled to provide an electrical signal or the like to the contact portion 110. For example, the integrated circuit 200 may deliver a command to the stimulator 230 to stimulate the cerebral cortex. One or more stimulators 230 may be included.

Meanwhile, the integrated circuit 200 may also serve to deliver external radio waves received by a separate wireless communication device to the integrated circuit through an electrode or an antenna, and to relay/transmit signals via a BCC antenna rather than directly receiving external radio waves.

The present invention may include an additional component to improve adhesion of the contact portion 110 to wet biological tissue. For example, the present invention may include a hydrophilic functional group bonded by performing hydrophilic surface treatment on a surface of an insulating layer formed on the contact portion, the insulating layer being a layer that blocks an electrode formed therein. The present invention may additionally include a hydrophobic microstructure formed on a portion of a surface of a lower support substrate on the opposite side.

In the contact portion 110 of the present invention, a surface directly contacting the cerebral cortex may be hydrophilically treated, and an opposite surface may be hydrophobically treated. In addition, it is possible to prevent the contacting surface from being flipped over.

The present invention may provide a method of measuring a signal of the brain and a method of stimulating the brain by using the system 10 including the cortical electrode and the wireless device.

The contact portion 110 may contact the cerebral cortex and receive an electrical signal or the like generated in the brain. Specifically, among the contact portion 110 of the implantable element 100, the graphene electrode layer serves to measure the electrical signal. The electrical signal may be delivered to the integrated circuit 200 connected to the transmission and reception portion 130 through a wiring layer, and, as will be described below, a brain signal received through a terminal may be visually provided.

Meanwhile, the transmission and reception portion 130 may be positioned in a space between the skull and the skin, receive external stimulation, and deliver the external stimulation to the cerebral cortex. Specifically, the transmission and reception portion 130 may receive external stimulation and deliver the external stimulation to the cerebral cortex.

As an example, the transmission and reception portion 130 may be connected to the integrated circuit 200, and the integrated circuit 200 may contact a surface of the skull or may be inserted into the skull.

Meanwhile, the system 10 including the cortical electrode and the wireless device according to the present invention may additionally include an amplifier. The amplifier may amplify an electrical signal measured by the electrode of the implantable element 100 of the present invention.

Meanwhile, although the system 10 including the cortical electrode and the wireless device according to the present invention has been described as being inserted into the brain, it is a matter of course that a position into which the implantable element 100 is inserted may be the spinal cord, a peripheral nerve, or the like within a range applicable by a person of ordinary skill in the art.

The system 10 including the cortical electrode and the wireless device according to the present invention may be used in vitro, ex vivo, or in vivo. A living body is an animal and may include a human, but may also be an animal excluding a human.

The system 10 including the cortical electrode and the wireless device according to the present invention provides a medical device. The medical device of the present invention may include the integrated circuit 200 connected to the transmission and reception portion 130 of the implantable element 100. Specifically, the implantable element 100 may be inserted through an opening of a skull region having a size of several mm² to several cm², the contact portion 110 of the implantable element 100 may contact the cerebral cortex, and the integrated circuit 200 may be connected to the transmission and reception portion 130 of the implantable element 100 and installed between the skull and the skin, and may preferably contact a surface of the skull or may be inserted into the skull. In this case, a portion of a lower end of the integrated circuit 200 may be exposed below the skull. Through the exposed lower end, the contact portion 110 of the implantable element 100 contacting the brain may be connected to the integrated circuit 200 through the connection portion 120.

In addition, the present invention may provide a cortical electrode and stimulation module further including a wireless communication device that delivers external radio waves to the integrated circuit 200. For example, as described above, transmission and reception may be performed only by a wireless module such as Bluetooth or a wireless communication network (Wi-Fi) in the implantable element 100 and the integrated circuit 200, but a wireless communication device may be additionally used for safer signal transmission and reception through body channel communication (BCC).

Specifically, external radio waves received through the wireless communication device are delivered to the integrated circuit through a BCC antenna. Accordingly, the BCC antenna may serve to relay/transmit signals via a BCC antenna rather than directly receiving external radio waves..

The wireless communication device is not limited as long as it can receive external stimulation and deliver the external stimulation to the integrated circuit, and may be a device in close contact with skin. For example, the wireless communication device may be a smart device worn on a wrist, waist, or the like.

The wireless communication device may communicate with an external terminal such as a computer or a smartphone through Bluetooth, a short-range wireless network (Wi-Fi), or the like, may provide various types of information to a user through a measured brain wave signal, and may deliver stimulation transmitted from the terminal to the integrated circuit 200. Additionally, the wireless communication device may also include a function of delivering information to a medical institution when an emergency signal is generated.

The present invention may also provide a method of stimulating a brain by inserting a cortical electrode into the brain of an animal. The animal may exclude a human. As described above, when a stimulation command is delivered from the outside, the stimulation command may be delivered to the integrated circuit 200 through an electrode or an antenna, and stimulation may be provided to the cerebral cortex through the implantable element 100 of the present invention.

For example, stimulation of the brain may be performed through the following process. When an abnormality such as a seizure is detected in brain waves measured through the implantable element 100, the integrated circuit 200 may receive the abnormality and provide the abnormality to the outside. When it is determined, based on the provided information, that stimulation needs to be applied from the outside, the stimulation may be delivered to the integrated circuit 200 and then delivered to the electrode of the implantable element 100 through the stimulator 230 or the like in the integrated circuit 200, so that electrical stimulation is delivered to the cerebral cortex. Through this process, an abnormal brain wave signal may be removed.

The present invention is intended to provide stimulation to the cerebral cortex through the implantable element 100. The stimulation is not limited thereto, but may be at least one selected from current stimulation, voltage stimulation, electric-field stimulation, and magnetic-field stimulation.

According to another embodiment of the present invention, the system 10 including the cortical electrode and the inductive-coupling-based wireless device may include the implantable element 100 and the integrated circuit 200 including a housing 300 and a stacked portion 260, and the integrated circuit 200 may be connected to the transmission and reception portion 130 to transmit and receive signals and may have a stacked structure.

Here, the integrated circuit 200 may transmit and receive information obtained through the implantable element 100. For example, electronic communication may be used.

In this case, the integrated circuit 200 may include a power device and may be capable of wireless communication.

In addition, the integrated circuit 200 may have a stacked structure. Specifically, the integrated circuit 200 may include devices such as a power device, a PCB, and an IC, and the devices may have the stacked structure to minimize volume. A detailed description will be provided below.

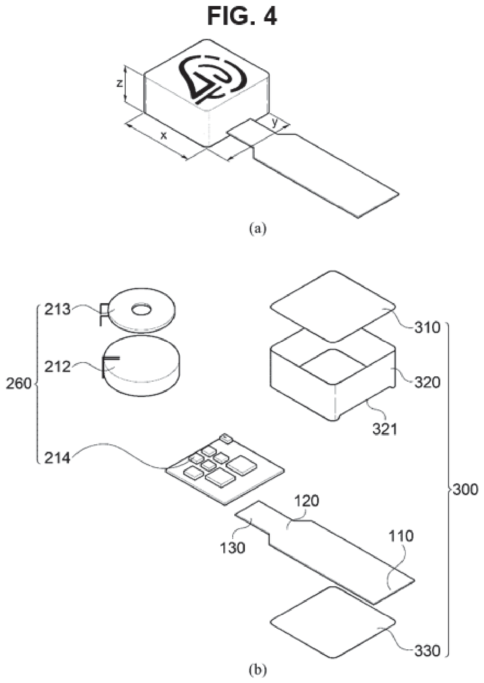

FIG. 4 is a view schematically illustrating a system including a cortical electrode and an inductive-coupling-based wireless device according to an embodiment of the present invention.

Referring to FIG. 4, according to another embodiment of the present invention, the housing 300 may have a structure having a horizontal dimension X of 25 mm, a vertical dimension Y of 25 mm, and a height Z of 10 mm, and may include an upper case 310 configured to protect an upper side of the stacked portion 260, a side case 320 having a height greater than a height of the stacked portion 260 and including a through portion 321 in at least a portion of one side thereof, and a lower case 330 formed below the stacked portion 260.

Here, the housing 300 may serve to protect devices included in the integrated circuit 200. Specifically, the housing 300 may accommodate the stacked portion 260 and a portion of the implantable element 100 therein, and may protect the accommodated devices from an outside of the wireless device so that the accommodated devices can electrically operate.

The upper case 310 may be formed above the stacked portion 260 and may protect the stacked portion 260.

The side case 320 may be formed on a side of the stacked portion 260 and may protect the stacked portion 260. In this case, the side case 320 protecting the stacked portion 260 may have a length greater than the height of the stacked portion 260.

The lower case 330 may be formed below the stacked portion 260 and may protect the stacked portion 260.

The respective components of the housing 300 formed as described above may be coupled to each other, and the upper case 310 and the lower case 330 may have the same size. In addition, the upper case 310 and the lower case 330 may have an area greater than an area of the stacked portion 260. Further, the side case 320 may have a height greater than the height of the stacked portion 260. Accordingly, the stacked portion 260 formed therein may be protected by the upper case 310, the side case 320, and the lower case 330.

Here, the housing 300 may be configured to surround the stacked portion 260 while forming at least a partial space. For example, when the stacked portion 260 is configured in a circular shape, the housing 300 may be configured in a rectangular shape so that at least a partial space is formed between the stacked portion 260 and the housing 300, specifically, between a side surface of the stacked portion 260 and the side case 320. Accordingly, a signal may be smoothly delivered to the outside through an antenna built in the PCB 214, or a signal received by the antenna from the outside may be smoothly delivered.

When the integrated circuit 200 integrated by the housing 300 serves to receive and process a signal generated in the cerebral cortex and obtained and transmitted by the implantable element 100, the housing 300 may protect electrical components inside the housing 300 from substances present in the body.

In addition, at least a portion of the integrated circuit 200 may be connected to the transmission and reception portion 130.

In this case, the side case 320 may include the through portion 321 having a shape in which a portion of the side case 320 is removed so that the transmission and reception portion 130 extending from the contact portion 110 may be inserted into the housing 300. The through portion 321 may have a shape corresponding to the transmission and reception portion 130 and may have a size greater than a size of the transmission and reception portion 130.

According to another embodiment of the present invention, the transmission and reception portion 130 may be formed such that at least a portion thereof passes through the through portion 321.

Here, the through portion 321 may have a shape in which a portion of the side case 320 is removed. Specifically, the through portion 321 may have a shape in which a portion is removed so as to form a gap that allows the transmission and reception portion 130 extending from the contact portion 110 to remain in a passed-through state.

In addition, the transmission and reception portion 130 may have a shape extending from the contact portion 110 and the connection portion 120, and accordingly, at least a portion of the transmission and reception portion 130, the connection portion 120, or the contact portion 110 may be formed in the through portion 321. Accordingly, the transmission and reception portion 130 may be formed to pass through the through portion 321 having the shape in which at least a portion is removed.

The side case 320 of the present invention may include an intrusion prevention portion configured to prevent intrusion of external substances through an empty space formed between the through portion 321 and the transmission and reception portion 130 after the transmission and reception portion 130 is inserted into the through portion 321. For example, the intrusion prevention portion may have a waterproof structure made of a material such as rubber or plastic.

According to another embodiment of the present invention, the upper case 310 may be formed of a synthetic resin material, preferably plastic, and the side case 320 and the lower case 330 may be formed of a metal material, preferably a titanium material.

Here, the upper case 310 may be formed of a synthetic resin so that a signal generated from the stacked portion 260 formed inside the housing 300 may be smoothly communicated.

In addition, the side case 320 and the lower case 330 may be formed of titanium.

When the side case 320 and the lower case 330 are formed of titanium, the side case 320 and the lower case 330 may not be affected by a subsequent procedure such as an MRI examination or a radiofrequency procedure.

Conversely, when the upper case 310 is formed of titanium, the upper case 310 may serve as a shield that blocks communication generated from the stacked portion 260, and thus, it may be preferable that the upper case 310 be formed of plastic.

According to another embodiment of the present invention, the stacked portion 260 may include a receiver coil 213, a battery 212, a PCB 214 on which an IC chip is mounted, and at least a portion of the transmission and reception portion 130.

Here, the receiver coil 213 may be a receiving coil and may receive power from the outside, and the battery 212 may be a flat cylindrical battery.

The IC may refer to a microscale complex electronic device or system in which many electronic circuit elements are inseparably coupled on a single substrate or in the substrate itself. Complex electronic components such as a transistor, a diode, a resistor, and a capacitor may be precisely manufactured and configured as a single electronic circuit in a small semiconductor. For example, rather than using individual semiconductors separately, thousands or tens of thousands of semiconductors may be collected and stacked on a plane of silicon. The IC may be mounted on the PCB 214 to be described below.

The PCB 214 may be a printed circuit board, and may be a board on which an IC is fixed to a surface of a printed wiring board and electronic circuits are configured by copper wiring lines between components. Here, the PCB 214 may include a component enabling wireless communication, that is, an antenna or the like.

Through the above-described configuration, information obtained from the implantable element 100 may be transmitted to the outside, and external information may be received.

In particular, this may be enabled wirelessly, so that the system can be used without additionally incising the scalp after a procedure.

According to another embodiment of the present invention, the transmission and reception portion 130 of the stacked portion 260 may be formed at a lowermost layer, and the stacked portion 260 may be stacked in the order of the transmission and reception portion 130, the PCB, the IC chip, the battery 212, and the receiver coil.

Each component may be the same as described above.

Here, the transmission and reception portion 130 may be connected to the connection portion 120 extending from the contact portion 110, and may be formed at a lowermost end of the stacked portion 260 to minimize stress on the implantable element 100. For example, when the transmission and reception portion 130 of the implantable element 100 is positioned at an uppermost end of the stacked portion 260, the transmission and reception portion 130 must be connected to the contact portion 110 of the implantable element 100 that contacts the cerebral cortex present below the skull. Accordingly, the implantable element 100 may be excessively bent, and stress may be concentrated on the implantable element 100 itself, causing damage thereto. To solve this, the transmission and reception portion 130 may be formed at the lowermost end of the stacked portion 260. That is, in the present embodiment, the transmission and reception portion 130 is disposed on a lower surface of the PCB in the stacked portion 260 and is connected to the PCB. In this case, the PCB may be configured such that an upper surface thereof faces the battery 212 and a lower surface thereof is connected to the transmission and reception portion 130.

Meanwhile, unlike the above-described embodiment, the transmission and reception portion 130 may be connected to an upper side of the PCB in the stacked portion 260. In this case, the PCB may be configured such that an upper surface thereof is connected to the transmission and reception portion 130 and a lower surface thereof faces the battery 212.

As another embodiment, the transmission and reception portion 130 may be connected to a side surface of the PCB in the stacked portion 260. In this case, the PCB may be configured such that an upper surface thereof faces the battery 212 and a lower surface thereof may be a lowermost side of the stacked portion 260.

As still another embodiment, the transmission and reception portion 130 may be connected to an upper side of the PCB in the stacked portion 260. In this case, the PCB may be the lowermost side of the stacked portion 260, and an upper surface thereof may be configured to be connected to the transmission and reception portion 130. In this case, the transmission and reception portion 130 may be disposed between the PCB and the battery 212.

In addition, the receiver coil 213 may be disposed at an uppermost side, thereby quickly and accurately receiving power from the outside. However, in the case of data, an antenna built in the PCB 214 is installed, and since the PCB 214 is positioned at a lower side of the stacked portion 260, wireless communication may not be smooth due to interference by the battery 212, the receiver coil 213, or the like installed on the upper side. To solve this, in the present invention, the housing 300 may be configured to surround the stacked portion 260 while forming at least a partial space. For example, when the stacked portion 260 is configured in a circular shape, the housing 300 may be configured in a rectangular shape so that at least a partial space is formed between the stacked portion 260 and the housing 300, specifically, between a side surface of the stacked portion 260 and the side case 320.

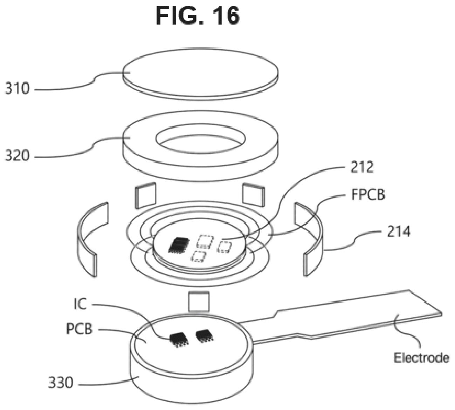

Meanwhile, as illustrated in FIG. 16, the integrated circuit may have a stacked structure. In this case, the PCB 214 may be disposed as in the above-described embodiment, and additionally, the PCB 214 may be configured to be disposed inside the side case 320. In this case, since some necessary components of the PCB 214 are disposed inside the side case 320, an area required for the PCB 214 can be reduced, thereby reducing an overall volume of the stacked portion 260. In this case, the housing 300 illustrated in FIG. 16 has a circular shape, and the PCB 214 disposed inside the side case 320 may be a flexible printed circuit board (FPCB).

According to another embodiment of the present invention, the battery 212 may be formed in a circular shape, and the housing 300 may be provided in a rectangular shape.

Here, the battery 212 may act as an interfering factor when generating a signal for information delivery. To minimize this, the battery 212 may be formed in a circular shape.

In addition, the housing 300 may be provided in a rectangular shape to optimize signal generation.

According to another embodiment of the present invention, each component of the stacked portion 260 may be electrically connected.

Here, the stacked portion 260 may be a device for reading and transmitting a signal of the cerebrum through the implantable element 100 and receiving and controlling an external signal.

In this case, the components of the stacked portion 260 may be electrically connected to transmit and receive information. Specifically, the transmission and reception portion 130, the PCB 214, the battery 212, and the receiver coil 213 may be electrically connected to each other, power received from the receiver coil 213 may be stored in the battery 212, and electrical energy stored in the battery 212 may be appropriately delivered to the PCB 214 and the transmission and reception portion 130.

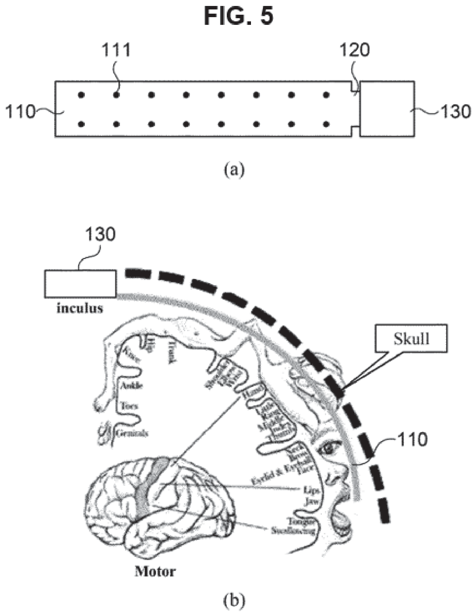

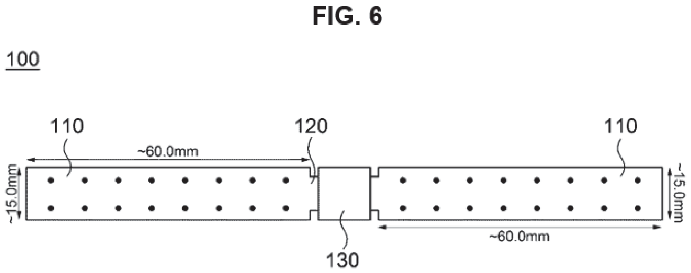

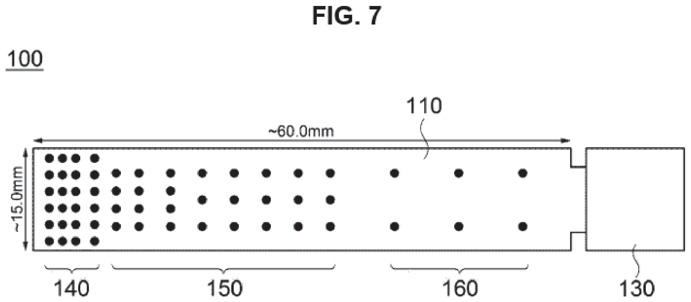

FIG. 5 is a view schematically illustrating an example of an implantable element 100 according to another embodiment of the present invention, FIG. 6 is a view schematically illustrating another example of the implantable element 100 according to another embodiment of the present invention, and FIG. 7 is a view schematically illustrating still another example of the implantable element 100 according to another embodiment of the present invention.

According to another embodiment of the present invention with reference to FIG. 5, the contact portion 110 may be formed such that a substrate extends in a longitudinal direction. In this case, the contact portion 110 may have, for example, a length of 60.0 mm in the longitudinal direction and a width of 15.0 mm in a width direction. Here, the longitudinal direction is a horizontal direction in FIG. 5, and the width direction is a vertical direction in FIG. 5.

Specifically, when the contact portion 110 contacts the cerebral cortex, the contact portion 110 may contact the motor cortex and may have a length capable of covering an outer surface of the motor cortex.

In this case, the contact portion 110 may have a rectangular shape, and at least one electrode 111 may be formed.

The electrode 111 may receive a signal generated in the brain and may deliver a signal to the brain by using electrical stimulation.

Here, the electrode 111 may be formed only on a front surface of the contact portion 110, or may be formed only on a rear surface thereof. In addition, the electrode 111 may pass through the contact portion 110 and be formed on both surfaces thereof.

According to another embodiment of the present invention with reference to FIG. 6, a plurality of contact portions 110 may be formed on both sides with respect to the transmission and reception portion 130.

Here, the contact portions 110 are connected to the transmission and reception portion 130 by the connection portions 120. For example, when the contact portions 110 are formed on both sides of the transmission and reception portion 130, the connection portions 120 may be formed on both sides, and each of the connection portions 120 may be connected to the corresponding contact portion 110.

In the case of such a form, the contact portions 110 can be formed in independent brain regions formed in both the left brain and the right brain through one transmission and reception portion 130.

According to another embodiment of the present invention with reference to FIG. 7, the contact portion 110 may include a left end portion 140, a middle portion 150, and a right end portion 160.

As illustrated in FIG. 7, when the transmission and reception portion 130 is formed on the right side and the contact portion 110 is formed on the left side, a side of the contact portion 110 may be defined as a left side, and a side of the transmission and reception portion 130 may be defined as a right side.

The left end portion 140 may be a region formed on the left side of the contact portion 110, and may be a region in which the largest number of electrodes 111 are formed.

The right end portion 160 may be a region closest to the integrated circuit 200, and may have the smallest number of electrodes 111.

The middle portion 150 may be a region connecting the left end portion 140 and the right end portion 160, and may have a number of electrodes that is between the number of electrodes of the left end portion 140 and the number of electrodes of the right end portion 160.

By concentrating electrodes in the left end portion 140 and forming the left end portion 140 to be heavier than the middle portion 150 and the right end portion 160, the left end portion 140 may be well seated on a surface of the brain.

In addition, when the contact portion 110 is attached to the cerebrum, nerves are densely distributed in a lower portion of the motor cortex on which the left end portion 140 is seated, and nerves are more densely distributed in portions corresponding to the eyes, nose, mouth, and the like than in portions corresponding to the feet, knees, and the like. Accordingly, many electrodes are required for stimulation or brain wave detection, thereby improving stimulation and data reception efficiency.

According to still another embodiment of the present invention, in the implantable element 100 contacting the cerebral cortex, the left end portion 140 may be formed at the leftmost side, and the implantable element 100 may be formed in the order of the left end portion 140, the middle portion 150, and the right end portion 160.

Specifically, from the integrated circuit 200, the implantable element 100 may be arranged in the order of the right end portion 160, the middle portion 150, and the left end portion 140.

By having such a configuration, the heaviest left end portion 140 is formed to be bent toward the cerebral cortex, and thus can be easily seated on the cerebral cortex.

According to still another embodiment of the present invention, electrode density may increase from the right end portion 160 toward the left end portion 140.

The electrode density may mean the number of electrodes present in a unit area. For example, a high density means that a large number of electrodes are present in the unit area.

The electrodes may have a certain weight, and when the electrode density increases, the weight may increase.

Accordingly, due to the increased weight, when the implantable element 100 is attached to the cerebrum, the left end portion 140 may be completely seated to the end without being lifted from the cerebral cortex.

In addition, when the implantable element 100 is attached to the cerebrum, a position of the left end portion 140 may be a position where many brain signals are generated, and signals may be accurately and quickly obtained by using a plurality of electrodes.

In addition, when a large number of electrodes are formed in the implantable element 100, an effect of broadly forming coverable lesion positions when the implantable element 100 is attached can be achieved.

According to still another embodiment of the present invention, the implantable element 100 may be formed of an elastic material.

Specifically, the implantable element 100 may be formed to be bent downward from the integrated circuit 200 toward the right end portion 160, the middle portion 150, and the left end portion 140 so as to be in close contact with the cerebrum. For example, the implantable element 100 may have a shape bent from an upper side of the brain toward a lower side thereof so that the implantable element 100 can be in close contact with the cerebrum.

The implantable element 100 may be formed to have elasticity and may be additionally bent at a predetermined angle when seated on the meninges.

According to still another embodiment of the present invention, the connection portion 120 connecting the contact portion 110 and the transmission and reception portion 130 may have a width smaller than a width of the contact portion 110 or the transmission and reception portion 130.

For example, the connection portion 120 having a width smaller than the width of the contact portion 110 may connect a right end of the rectangular contact portion 110 to the transmission and reception portion 130. Here, the connection portion 120 may serve to physically connect the contact portion 110 and the transmission and reception portion 130 while enabling electrical transfer.

The connection portion 120 having such a form can minimize waste of material and improve flexibility.

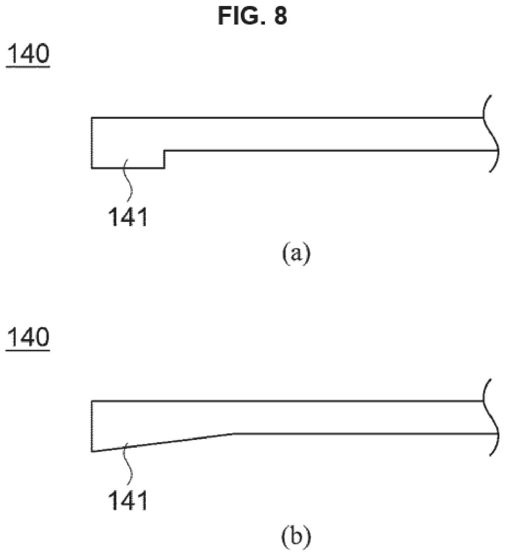

FIG. 8 is a view schematically illustrating an example of the left end portion 140 according to another embodiment of the present invention.

According to still another embodiment of the present invention with reference to FIG. 8(a), an end of the left end portion 140 may include a stepped portion 141.

Here, the stepped portion 141 may be formed to have a predetermined weight so that all end portions of the implantable element 100 can be seated on the brain when the implantable element 100 comes into contact with the meninges. For example, when the stepped portion 141 has a predetermined weight, the implantable element 100 can be easily moved in a direction of gravity or in a direction in which a user shakes the implantable element 100 when the implantable element 100 is seated on the meninges due to the weight of the stepped portion 141, and thus the elasticity of the implantable element 100 due to movement can be utilized to the maximum extent.

According to still another embodiment of the present invention, the stepped portion 141 may be provided in a rectangular shape.

For example, the stepped portion 141 having a thickness of the implantable element 100 may be additionally formed at an end of the left end portion 140 so as to contact the meninges.

According to still another embodiment of the present invention with reference to FIG. 8(b), the stepped portion 141 may have a gradient structure in which a thickness thereof becomes greater toward the end of the left end portion 140.

The gradient structure may be a structure for minimizing a space between the meninges and the implantable element 100 when the implantable element 100 is seated on the meninges.

For example, in a state in which the implantable element 100 is seated on the meninges due to the weight of the stepped portion 141, the gradient structure may allow a portion of the meninges to be flexibly fitted to a shape of the stepped portion 141.

According to still another embodiment of the present invention, a thickness of an end of the right end portion 160 may be 16 µm, and a thickness of an end of the left end portion 140 may be 18 µm to 20 µm.

Here, the end of the right end portion 160 may be an end on a side of the transmission and reception portion 130.

In addition, the end of the left end portion 140 may be an end opposite to the transmission and reception portion 130.

Due to the end of the left end portion 140 being formed thicker than the end of the right end portion 160, when the implantable element 100 is seated on the cerebrum, the implantable element 100 can be well seated on an irregular shape of the cerebrum.

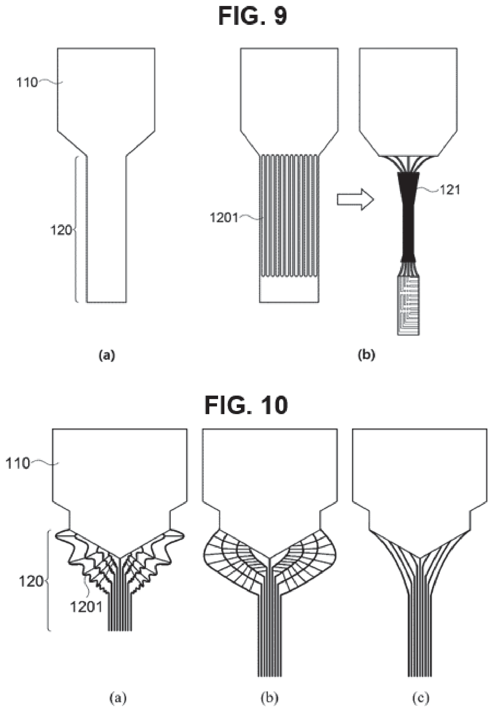

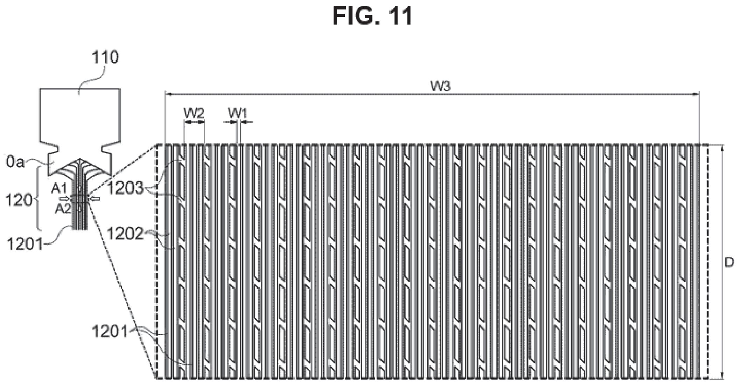

FIG. 9 is a view schematically illustrating an example of the connection portion 120 according to another embodiment of the present invention, FIG. 10 is a view schematically illustrating another example of the connection portion 120 according to another embodiment of the present invention, and FIG. 11 is a view schematically illustrating still another example of the connection portion 120 according to another embodiment of the present invention.

According to still another embodiment of the present invention with reference to FIG. 9, the connection portion 120 may include a plurality of wire-shaped substrates 1201. In the present embodiment, the contact portion 110 may be formed as a large-area multi-channel surface electrode. As the contact portion 110 is formed to have a larger number of channels, when the connection portion 120 connected to the transmission and reception portion 130 is manufactured in a film-like planar form, a width of the planar form becomes large, which has a disadvantage in that the scalp must be widely incised during surgery. Accordingly, in the present invention, the connection portion 120 is provided such that the wire-shaped substrates 1201 have a bundle form, and thus, when the contact portion 110, which is a large-area flexible electrode, is inserted into the cerebral cortex and then connected to an external measurement terminal, a wide incision of the scalp is not required.

Here, the plurality of wire-shaped substrates may be obtained by separating a plate-shaped substrate into a plurality of wire shapes. In this case, wiring lines may be formed on the respective plurality of wire-shaped substrates so as to be separated for each wiring line.

In addition, the plurality of wire-shaped substrates may be provided in different colors, respectively. This may be for easily distinguishing a plurality of wires according to the respective wiring lines.

The connection portion 120 having the plurality of wire-shaped substrates may form a bundle by using a shrink tube 121, which will be described below. This may increase stiffness.

The connection portion 120 having the plurality of wire-shaped substrates may be compressed by using the shrink tube 121.

The connection portion 120 provided in a thin wire shape may be connected by passing through an incised skin region. In this case, breakage may occur due to weak stiffness of the connection portion 120. To prevent this, the plurality of wire-shaped substrates may be bundled into one.

The shrink tube 121 may be formed to have predetermined stiffness so as to bundle the plurality of wire-shaped substrates into a single line shape. When the bundled plurality of wire-shaped substrates pass through the skull, the bundled plurality of wire-shaped substrates can have stiffness while minimizing volume.

According to still another embodiment of the present invention, the shrink tube 121 may coat the bundled plurality of wire-shaped substrates by using a flexible material.

For example, the bundled plurality of wire-shaped substrates may be coated with silicone from an outer side.

By having such a form, the connection portion 120 may have stiffness sufficient to pass through the skin region, and connection can be performed easily.

In addition, the connection portion 120 may be formed to have a serpentine structure to absorb strain applied to a portion connected to the transmission and reception portion 130, and this portion may be coated with soft rubber or, for example, silicone.

According to still another embodiment of the present invention with reference to FIG. 10, at least a portion of the connection portion 120 may have a serpentine structure.

Here, the serpentine structure may be a structure in which the wire-shaped substrate 1201 has a predetermined shape.

For example, the wire-shaped substrate 1201 may have a serpentine shape while becoming wider toward the contact portion 110.

This is because Parylene is used at 6% to 7% or less and gold is used at 1% or less in a material used to manufacture the implantable element 100, and thus stretchability is not high, and the electrode structure also adversely affects stretchability. Therefore, when a process of bundling the wire-shaped substrates 1201 into one and wrapping them with silicone, the shrink tube 121, or the like is performed, strain generated during the process is concentrated in a specific region. In this case, when the strain is concentrated on a portion having weak durability, there is a risk of breakage or damage. Accordingly, in order to concentrate the strain concentrated in the specific region on a portion having relatively high durability, the connection portion 120 may be configured with the wire-shaped substrates 1201, and at least a portion of the wire-shaped substrates 1201 may be formed to have the serpentine structure. Since the effective stiffness of the serpentine structure of the connection portion 120 is significantly smaller than that of other materials, the strain concentrated in the specific region may be concentrated on the connection portion 120.

In particular, at a portion where the wire-shaped substrates 1201 are connected to the contact portion 110, when the wire-shaped substrates 1201 are bundled into one, an inward pulling strain may be generated in an outermost wire-shaped substrate 1201. Accordingly, the outermost wire-shaped substrate 1201 may be configured to have the most serpentine shape.

When the wire-shaped substrates 1201 are bundled into one, the connection portion 120 formed as described above may minimize strain as a serpentine region is unfolded and a length thereof increases.

According to still another embodiment of the present invention, the wire-shaped substrate 1201 of the connection portion 120 may have a serpentine shape only in a portion close to the contact portion 110.

Strain generated when the wire-shaped connection portion 120 is bundled into one occurs in a portion close to the contact portion 110, and among the wire-shaped substrates 1201, the strain may be strongly generated in an outer wire-shaped substrate 1201. Accordingly, the wire-shaped substrate 1201 may be provided to have a serpentine shape only in a portion of the connection portion 120 close to the contact portion 110.

According to still another embodiment of the present invention, a region of the contact portion 110 connected to the connection portion 120 may be provided in a 'V'-shape.

This may be for maximally securing a length and minimizing generated strain when the wire-shaped substrates 1201 having the serpentine structure of the connection portion 120 are connected to the contact portion 110. For example, an inner wire-shaped substrate 1201 may be formed to be the shortest, and the wire-shaped substrates 1201 may be formed such that lengths thereof become longer toward an outer side.

According to still another embodiment of the present invention with reference to FIG. 11, the wire-shaped substrates 1201 of the connection portion 120 may have 64-channel wiring lines. Specifically, in the wire-shaped substrates 1201, wiring lines 1202 are formed on the substrates, 20 wire-shaped substrates 1201 each including three wiring lines 1202 are formed, and one wire-shaped substrate 1201 including two wiring lines 1202 is formed on each of both sides of the 20 wire-shaped substrates 1201, so that a total of 64 wiring lines 1202, that is, 64 channels of wiring lines 1202, may be formed. In this case, a width W1 of the wiring line 1202 may be 10 µm to 30 µm, preferably 15 µm, and a width W2 of one wire-shaped substrate 1201 may be 50 µm to 200 µm, preferably 85 µm. Preferably, a total width W3 of the wire-shaped substrates 1201 may be 2.24 mm. In this case, a thickness D of the wire-shaped substrate 1201 may be 15 µm or more. The number of wiring lines 1202 that may be formed on one wire-shaped substrate 1201 may be changed according to design. In this case, a substrate 1203 connecting the wire-shaped substrates 1201 to each other may be formed, and the connection substrate 1203 may connect the wire-shaped substrates 1201 at an angle of 45 degrees. If the connection substrate 1203 is connected at an angle of 90 degrees, stiffness in the X-axis direction increases and may hinder bundling of the wire-shaped substrates 1201. Accordingly, the connection substrate 1203 may connect the wire-shaped substrates 1201 at an angle of 45 degrees. This angle of 45 degrees can prevent tearing of the wire-shaped substrate 1201 due to high stiffness in the X-axis direction when the connection portion 120 is forcibly bundled while being wrapped with the shrink tube 121, silicone, or the like. That is, when the connection substrate 1203 is connected at an angle of 90 degrees, tearing of the wire-shaped substrate 1201 may be induced due to high stiffness in the X-axis direction, whereas when the connection substrate 1203 is connected at an angle of 45 degrees, such tearing can be prevented.

In addition, a length in a Y-axis A2 direction of the wire-shaped substrate 1201 of the connection portion 120 may be formed to be 10 times or more a length in an X-axis A1 direction. When the length in the Y-axis A2 direction of the wire-shaped substrate 1201 is formed to be long, the wire-shaped substrates 1201 may be induced to be easily bundled together in the X-axis A1 direction while elastic energy accumulated in the Y-axis A2 direction is made zero. This may also cause strain to be concentrated on the connection portion 120.

The thinly formed wire-shaped substrate 1201 may have flexibility when bundled in the X-axis A1 direction, but problems such as tearing or breakage may occur accordingly.

Accordingly, according to still another embodiment of the present invention, a region of the contact portion 110 connected to the wire-shaped substrates 1201 may be provided in an 'A'-shape 110a.

Here, the 'A'-shape 110a may be a cone shape having a pointed center, and the wire-shaped connection portion 120 may be connected to an inside of the 'A'-shape 110a.

The contact portion 110 having a lower end provided in the 'A'-shape 110a may reduce strain so as to prevent tearing due to pulling of the wire-shaped substrates 1201 when the wire-shaped substrates 1201 of the connection portion 120 are bundled in the X-axis direction. The wire-shaped connection portion 120 formed as described above has an effect of minimizing a width of an incision in the scalp when the large-area implantable element 100 is connected to an external measurement terminal after insertion.

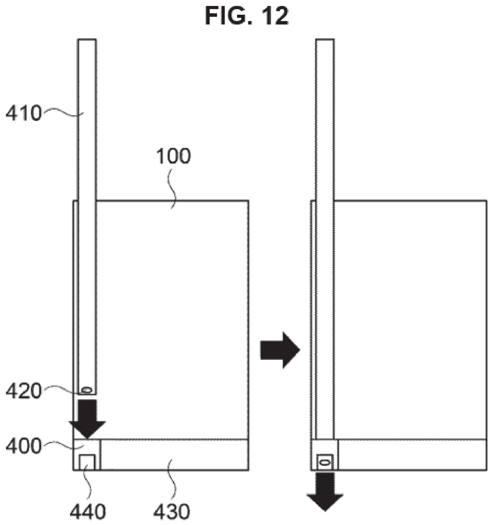

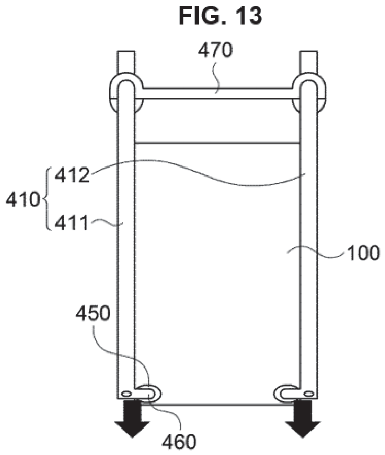

FIG. 12 is a view schematically illustrating an example of a system including a guide portion 410 according to another embodiment of the present invention, and FIG. 13 is a view schematically illustrating a guide portion 410 including a fixing member 470 according to another embodiment of the present invention.

According to still another embodiment of the present invention with reference to FIG. 12, the system 10 including the cortical electrode and the inductive-coupling-based wireless device may be a system for inserting the cortical electrode into a surface of the cerebral cortex, and may include a pocket portion 400 provided in a pocket shape and having a predetermined space formed therein, and a guide portion 410, which is a guide structure insertable into the pocket portion 400 and at least partially provided in a rigid rod shape so as to push the cortical electrode into the surface of the cerebral cortex. The system 10 may include the implantable element 100 that contacts the surface of the cerebral cortex and measures a signal generated in the brain or delivers external stimulation to the brain.

Here, the cortical electrode may mean the implantable element 100. For example, the cortical electrode may directly contact the cerebral cortex and serve to receive a signal of the brain or deliver information received from the outside to the brain.

In addition, the implantable element 100 may be the above-described implantable element 100 and may be a substrate, that is, an electrode, made of a flexible material, but is not limited thereto.

In addition, the implantable element 100 may be inserted by opening the skull so as to contact the cerebral cortex. In this case, in order to minimize an area of the skull to be opened, the implantable element 100 may be formed in the above-described form of the implantable element 100, and the skull may be opened to a width into which the implantable element 100 can be inserted, and the implantable element 100 may be inserted by using the pocket portion 400 and the guide portion 410. A detailed description will be provided below.

The pocket portion 400 may be a predetermined space into which the guide portion 410 is inserted. For example, the pocket portion 400 may be formed at an end of the implantable element 100 in a longitudinal direction, and may be formed at any one of one side, the other side, and a middle side in a width direction, but may preferably be formed at the middle side. The pocket portion 400 may be formed in a state in which a front side thereof is open so that a lens of the camera 420 formed in the guide portion 410 can look forward during insertion. In this case, the pocket portion 400 does not have a form in which the entire front side is open, but has a form in which at least a portion thereof, preferably only a central portion thereof, is open. Accordingly, it is possible to prevent the guide portion 410 inserted into the pocket portion 400 from passing through the pocket portion 400 while securing a forward field of view. That is, the pocket portion 400 may include an imaging hole 440 having a form opened in at least a portion thereof. In addition, the implantable element 100 may be formed of a flexible material, and only a portion in which the pocket portion 400 is formed may be formed of a material having high stiffness. When an operation of pushing the implantable element 100 is performed, it is possible to minimize damage to the cerebral cortex and, at the same time, prevent the implantable element 100, which has lower stiffness than the guide portion 410, from being folded or torn.

That is, according to another embodiment of the present invention, the implantable element 100 may include a reinforcing plate 430 to prevent an end of the implantable element 100 from being torn.

Here, the reinforcing plate 430 may be formed by overlaying a material having high stiffness on a portion of the implantable element 100 in which the pocket portion 400 is formed. Accordingly, while preventing an end of the implantable element 100 pushed by the guide portion 410 from being folded or torn, the entire implantable element 100 may be seated on the cerebral cortex.

The guide portion 410 may be formed to be inserted into the pocket portion 400, and after being inserted, may insert the implantable element 100 into the inside of the skull along the cerebral cortex by pushing the implantable element 100. In this case, the guide portion 410 may push the pocket portion 400 while being inserted into the pocket portion 400, thereby pushing the entire implantable element 100. In addition, the guide portion 410 may be formed of a material stiffer than the implantable element 100 and may guide the implantable element 100.

In addition, the guide portion 410 may include the camera 420 at an end portion that contacts the pocket portion 400. Here, the camera 420 may be an endoscopic camera 420. The camera 420 may be a means for checking the inside of the opened cerebrum.

In addition, a wire connected to the camera 420 may be formed inside the guide portion 410.

The camera 420 formed in the guide portion 410 may capture an image of a front side of the pocket portion 400 being pushed into the inside. In this case, illumination for imaging may be formed, or, conversely, the camera 420 may be an infrared camera that does not require light.

According to still another embodiment of the present invention, the pocket portion 400 may form the imaging hole 440 having a form opened in at least a portion thereof so that imaging by the camera 420 can be performed while the pocket portion 400 is pushed in by the guide portion 410.

Here, the imaging hole 440 may have a size sufficient to allow the camera 420 to capture an image, and may be formed at a center of a front side of the pocket portion 400. In addition, a portion of the guide portion 410 other than a portion corresponding to the camera 420 may have a closed structure so that the guide portion 410 does not pass therethrough.

Accordingly, the guide portion 410 can push the implantable element 100 into the cerebral cortex while performing imaging.

According to still another embodiment of the present invention with reference to FIG. 13, the implantable element 100 may include engagement grooves 450 at both lower ends thereof, and the guide portion 410 may include at least one engagement protrusion 460 having a protruding shape so as to be engageable with the engagement grooves 450. Here, the guide portion 410 may include a first guide structure 411 and a second guide structure 412.

Here, the engagement grooves 450 may be formed at lower ends of the implantable element 100. Specifically, the engagement grooves 450 may be in the form of recessed grooves or through-holes respectively formed on both sides at the lower ends of the implantable element 100 toward a bottom surface.

In addition, the engagement protrusion 460 may be formed to be smaller than the engagement groove 450. For example, when the engagement protrusion 460 is formed to be larger than the engagement groove 450, the engagement protrusion 460 may not be engageable with the engagement groove 450, and this may be prevented.

In addition, the guide portion 410 may include a single engagement protrusion 460, but is not limited thereto, and may include a plurality of engagement protrusions 460. This is because, when the single engagement protrusion 460 is engaged with the engagement groove 450 and pushes the implantable element 100, the engagement protrusion 460 may be separated therefrom, and such separation needs to be prevented. Through a configuration in which the engagement grooves 450 and the engagement protrusions 460 are formed on both sides of the implantable element 100, the implantable element 100 can be held from both sides, thereby preventing separation caused by movement of the implantable element 100.

According to still another embodiment of the present invention with reference to FIG. 13, the guide portion 410 may include, at a middle side thereof, a fixing member 470 configured to fix a plurality of guide portions 410 such that the plurality of guide portions 410 are spaced apart from each other by a predetermined distance in a width direction.

Here, the fixing member 470 may prevent each of the first and second guide structures 411 and 412 from widening or narrowing in the width direction, and may allow the first and second guide structures 411 and 412 to be spaced apart from each other by a predetermined interval.

In this case, the connected first and second guide structures 411 and 412 may be formed to have the same length from a connected portion to a lower end, and the fixing member 470 may be formed to have the same width as the implantable element 100. When the guide portion 410 pushes the implantable element 100 after being engaged with the engagement grooves 450, both sides of the implantable element 100 can be pushed equally. In addition, it is possible to prevent a force from being biased to one side and causing the engagement protrusion 460 to be separated from the engagement groove 450, and the implantable element 100 can be inserted smoothly without wrinkling during an insertion process.

The engagement protrusions 460 may be formed to protrude in the width direction from each of the first and second guide structures 411 and 412, and may be formed in a ' 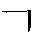 '-shaped hook form. In this case, the fixing member 470 may be formed to be rotatable when connected to each guide portion 410, so that engagement and release of the engagement protrusion 460 with respect to the engagement groove 450 can be implemented by rotation.

According to another embodiment of the present invention, the engagement groove 450 may be formed to have a size smaller than or equal to a size of the engagement protrusion 460.

The engagement protrusion 460 formed in the guide portion 410 is engaged with the engagement groove 450 and serves to push the implantable element 100.

In this case, in order to prevent the engagement protrusion 460 from being easily separated from the engagement groove 450, the engagement protrusion 460 may be formed to be smaller than the engagement groove 450 or may have a specific shape.

Specifically, the engagement groove 450 may be provided in a circular shape or a rectangular shape, and the engagement protrusion 460 may have a ' 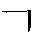 '-shaped hook form or a ' 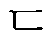 '-shape in which an opening of the ' 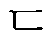 '-shape faces downward. Alternatively, a protruding member facing forward may be additionally provided at a lower end of the ' 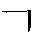 '-shaped hook, so that stronger fixing force can be provided when the implantable element 100 is pushed forward.

According to another embodiment of the present invention, at least one of the first and second guide structures 411 and 412 may have a camera 420 formed at a lower end thereof.

The camera 420 may capture an image of the cerebral cortex inside the skull, and may guide an operator so that the implantable element 100 can be positioned at an accurate position without damaging the cerebrum while the first and second guide structures 411 and 412 made of rigid materials on both sides are pushed in.

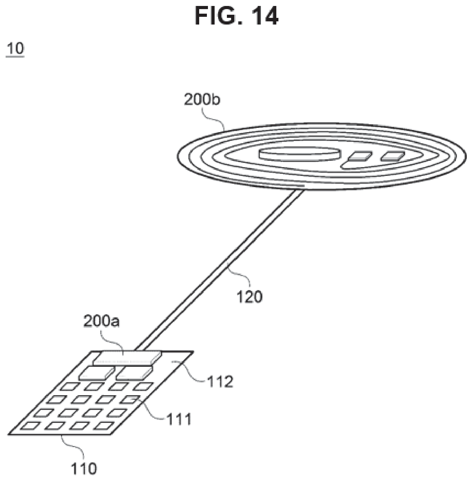

FIG. 14 is a view schematically illustrating another example of a system according to another embodiment of the present invention, and FIG. 15 is a view schematically illustrating another example of a system according to another embodiment of the present invention. In FIG. 15, (a) schematically illustrates the system according to another embodiment of the present invention as a block diagram, (b) is a cross-sectional view of a system according to an embodiment of the present invention, and (c) is a cross-sectional view of a system according to another embodiment of the present invention.

According to another embodiment of the present invention with reference to FIG. 14 or FIG. 15, the contact portion 110 of the implantable element 100 of the system may be integrated up to a brain wave collection device 200a for collecting brain wave information, that is, a converter 291, a chip controller 240, and a recorder 220, of the integrated circuit 200.

For example, when the implantable element 100 is seated on the cerebral cortex and receives a signal of the cerebrum, the brain wave collection device 200a may serve to store the signal and may convert analog information into digital information.

Specifically, the brain wave collection device 200a may be provided in the contact portion 110 of the implantable element 100. In the contact portion 110, an electrode array 111 may be disposed on a substrate 112 at a portion far from the connection portion 120, that is, a distal portion, and the brain wave collection device 200a, that is, an amplifier 290, a converter 291, a chip controller 240, and the recorder 220, may be included at a portion close to the connection portion 120, that is, a proximal portion.

When the brain wave collection device 200a is directly exposed to the outside, the brain wave collection device 200a may fail due to moisture.

To prevent this, waterproof packaging may be performed on the contact portion 110 of the implantable element 100. For example, the brain wave collection device 200a formed on one side of the implantable element 100 may be formed to be surrounded by a waterproof material, such as plastic, rubber, silicone, or the like. In addition, the entire implantable element 100 may be coated.

According to another embodiment of the present invention, the implantable element 100 may include the contact portion 110 including an electrode 111 configured to contact a surface of the cerebral cortex and measure a signal generated in the brain or deliver external stimulation to the brain, and the brain wave collection device 200a connected to the electrode 111 so as to be capable of exchanging information with the electrode 111, that is, an amplifier 290, a converter 291, a chip controller 240, and a recorder 220. The system may include the integrated circuit 200 configured to exchange information delivered from the implantable element 100 with the outside and receive power, and the implantable element 100 may include the connection portion 120 connecting the contact portion 110 and the integrated circuit 200.

As described above, the contact portion 110 of the implantable element 100 may include the amplifier 290, the converter 291, the chip controller 240, and the recorder 220. Meanwhile, the chip controller 240 may not be disposed only in the contact portion 110 of the implantable element 100, but may be excluded from the contact portion 110 and disposed outside the skull. In addition, the contact portion 110 may include the electrode 111 and may receive an analog signal of the cerebrum while being in contact with the cerebral cortex. In this case, the contact portion 110 may include at least one electrode 111.

Analog information received through the electrode 111 of the contact portion 110 may be converted into digital information by using the brain wave collection device 200a and stored. The digital information stored as described above may be transmitted to the integrated circuit 200 through wiring lines of the connection portion 120, and the transmitted information may be transmitted to the outside of the body through wireless communication. Conversely, information received from the outside of the body may be converted into analog information and delivered to a stimulator, and the stimulator may deliver stimulation to the cerebrum by applying a signal to the electrode 111 of the contact portion 110.

The contact portion 110 and the integrated circuit 200 may be connected by the connection portion 120. The connection portion 120 includes at least one wiring line, and may supply power from the integrated circuit 200 to the contact portion 110 through the wiring line or may transmit and receive information through the wiring line.

According to another embodiment of the present invention, the connection portion 120 of the implantable element 100 may be configured with only two wiring lines and connected to the integrated circuit 200. The two wiring lines may be the power line 295 and the data line 296, and the power line 295 may be divided into a VDD wiring line and a ground wiring line.

The power line 295 may be a wiring line for applying power.

In addition, the data line 296 may be a wiring line for transmitting and receiving information. Here, the data line 296 may include a signal ground wiring line, and may also be configured with a plurality of data lines.

As described above, according to the present invention, the contact portion 110 of the implantable element 100 is integrated up to the brain wave collection device 200a that collects brain wave information, so that the connection portion 120 connected to the integrated circuit 200 has only two wiring lines, that is, the power line 295 and the data line 296. Accordingly, a thickness of the connection portion 120 can be minimized.

In this case, the two wiring lines 295 and 296 may further include a housing surrounding the outside thereof.

According to another embodiment of the present invention, the integrated circuit 200 may include an inductive-coupling-based wireless device 200b.

The wireless device 200b may include an on-chip coil 292, a current converter 293, a power management circuit 294, and a wireless chip 250. Here, the current converter 293 may convert alternating current received from the outside through the on-chip coil 292 into direct current and then deliver the direct current to the power management circuit 294. The power management circuit 294 may be connected to the chip controller 240 of the contact portion 110 of the implantable element 100 through the power line 295, and the wireless chip 250 may be connected to the recorder 220 of the contact portion 110 of the implantable element 100 through the data line 296. Of course, the chip controller 240 is connected to the recorder 220.

Accordingly, the implantable element 100 may itself store a signal of the cerebrum and transmit the stored information to the integrated circuit 200 attached to the skull. The integrated circuit 200 may transmit the stored information by using the wireless communication device 250, that is, the wireless chip, or may wirelessly receive power from the outside. Here, when the wireless chip is connected to the recorder 220, an encoder may be provided therebetween.

According to another embodiment of the present invention, the contact portion 110 may include at least one electrode 111, and the chip controller 240 may form the same number of channels as the number of electrodes.

Here, the electrode 111 may contact the cerebral cortex to measure brain waves or deliver a signal to the cerebrum. In this case, a large number of electrodes may be formed, and the electrodes may be formed to read various signals in order to read all signals generated in the cerebrum.

In addition, channels corresponding to the number of brain waves generated in the cerebrum may be formed. In this case, electrodes for reading brain waves may form channels corresponding to the number of brain waves.

Since the channels form a bundle of wires, the shorter the channels are, the more information errors can be minimized and waste of materials can be reduced. For example, when information obtained from the contact portion 110 is transmitted through 64 channels, if the chip controller 240 and the recorder 220 are formed outside the implantable element 100, 64-channel wires need to be connected between the implantable element 100 and the external chip controller 240 and recorder 220. As a result, durability may be weakened and material waste may occur. See FIG. 15(b).

According to another embodiment of the present invention, the implantable element 100 and the integrated circuit 200 may be connected by two lines 295 and 296. See FIG. 15(c).

When two lines, that is, two channels, are formed, the lines may include a voltage VDD line, a ground line, and a data line therein. Since the number of lines is smaller than when a plurality of lines are formed, volume can be minimized, and an opening area can be minimized when the integrated circuit 200 on the upper side of the skull and the implantable element 100 contacting the cerebral cortex are connected.

According to another embodiment of the present invention, the implantable element 100 may convert analog information collected from the contact portion 110 into digital information by using the chip controller 240 and transmit the digital information to the integrated circuit 200 by using the connection portion 120.

Here, the analog information may be short-wave information received from the cerebrum. The chip controller 240 may convert the analog information into a digital signal, and the recorder 220 may store the converted digital signal. The stored digital information may be transmitted to the integrated circuit 200, and the integrated circuit 200 and an external transmission/reception device may transmit the digital signal.

According to another embodiment of the present invention, when waterproof-coating the contact portion 110, the system may waterproof-coat the chip controller 240 and the recorder 220 together.

Here, the coating may be performed because the implantable element 100 contacting the cerebral cortex may be wetted by liquid on a surface of the cerebrum. In this case, the coating may be performed to prevent an electrical connection from being discharged or short-circuited due to moisture. The coating may be performed by using rubber, silicone, or the like, and preferably may be a silicone coating.

In this case, the implantable element 100 may be positioned together with the chip controller 240 and the recorder 220, and all of them may be coated.

According to another embodiment of the present invention, the implantable element 100 including a plurality of channels may be formed inside the skull, and the implantable element 100 may be connected, through the connection portion 120, to the integrated circuit 200 formed outside the skull.

The implantable element 100 including the chip controller 240 and the recorder 220 may be formed to contact the cerebral cortex inside the skull. The implantable element 100 may receive a signal generated from the cerebrum, convert the signal into a digital signal, and store the digital signal. In order to transmit information converted into the digital signal to the outside, the digital information may be transmitted to the integrated circuit 200. The digital information requires only a minimum number of wiring lines, and accordingly, the connection portion 120 connecting the implantable element 100 and the integrated circuit 200 may have a very thin form. Accordingly, an opened portion of the skull can be formed to be very narrow.

In addition, the contact portion 110 that receives an analog signal obtains information by using a plurality of electrodes, and in this case, a plurality of channels are used. The channels should be formed with separate wiring lines, respectively, and accordingly, a very large number of wiring lines are formed.

The wiring lines should have the same wiring lines until the analog signal is converted into a digital signal. That is, the wiring lines may have a form including a plurality of wiring lines until the analog signal obtained from the contact portion 110 passes through the chip controller 240. The plurality of wiring lines occupy a large volume, and when the implantable element 100 is inserted or used, the volume of an opened skull may increase, and damage to the body may occur. Accordingly, in order to minimize a size of the connection portion 120, the connection portion 120 is configured to have only two wiring lines. To this end, not only the electrode 111 is disposed in the contact portion 110 of the implantable element 100, but also the chip controller 240 and the recorder 220 are disposed and integrated together in the contact portion 110, rather than being disposed in the integrated circuit 200. Accordingly, a volume of the connection portion 120 can be minimized.

The present invention may have the following first embodiment.

The present invention includes an implantable element including a contact portion configured to measure a signal generated in the brain or deliver stimulation to the brain, a transmission and reception portion configured to transmit, to the outside, a signal received from the contact portion or deliver, to the contact portion, a signal indicating the stimulation, and a connection portion connecting the contact portion and the transmission and reception portion. The contact portion is formed to have a length in a longitudinal direction and includes at least one electrode, and the largest number of electrodes are formed in a portion of the contact portion farthest from the connection portion.

Specifically, a plurality of contact portions may be formed on both sides of the transmission and reception portion.

Specifically, the contact portion may include a right end portion in which at least one electrode is formed, a left end portion formed at an end of the contact portion, and a middle portion connecting the left end portion and the right end portion. The electrode density may increase in the order of the right end portion, the middle portion, and the left end portion.

Specifically, the contact portion may be formed of an elastic material, and the left end portion may form a stepped portion formed such that a thickness of at least a portion of a left end is different.

Specifically, the stepped portion may have a rectangular structure configured to have a predetermined thickness by a predetermined distance from an end of the left end portion toward the right end portion.

Specifically, the stepped portion may have a gradient structure formed such that a thickness thereof decreases by a predetermined distance from the end of the left end portion toward the right end portion.

Specifically, the connection portion may be formed such that at least one wire-shaped substrate has a bundle form.

Specifically, the connection portion may include a shrink tube configured to fix at least one wire-shaped substrate.

Specifically, the connection portion may be configured such that at least a portion of at least one wire-shaped substrate has a serpentine structure.

The present invention may have the following second embodiment.

The present invention provides a cortical electrode system including an implantable element configured to contact a surface of the cerebral cortex and measure a signal generated in the brain or deliver external stimulation to the brain, the cortical electrode system including a pocket portion provided in a pocket shape on one side of the implantable element and forming a predetermined space therein, wherein the pocket portion is formed such that a rod-shaped guide portion for inserting the implantable element into the surface of the cerebral cortex is inserted therein.

Specifically, the implantable element may include a reinforcing plate extending from one side of the pocket portion and configured to prevent tearing caused by insertion of the guide portion.

Specifically, the guide portion may be an endoscope, and the pocket portion may include an imaging hole having a form opened in at least a portion thereof so that the guide portion can be inserted and imaging can be performed.

Specifically, the implantable element may include a plurality of pocket portions and may include a plurality of guide portions respectively inserted into the plurality of pocket portions.

The present invention provides a cortical electrode system including an implantable element configured to contact a surface of the cerebral cortex and measure a signal generated in the brain or deliver external stimulation to the brain. The implantable element includes engagement grooves respectively provided on both sides of one end thereof and engagement protrusions each having a protruding shape so as to be engageable with the respective engagement grooves. The cortical electrode system includes rod-shaped first and second guide structures configured to insert the implantable element into the surface of the cerebral cortex.

Specifically, the first and second guide structures may include, at a middle side thereof, a fixing member configured to fix the first and second guide structures such that the first and second guide structures are spaced apart from each other by a predetermined distance.

Specifically, the engagement protrusion may be formed to protrude in a ' 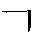 '-shape from the first or second guide structure so as to be inserted into the engagement groove.

Specifically, at least one of the first and second guide structures may be an endoscope.

The present invention may have the following third embodiment.

The present invention includes an implantable element including a contact portion configured to measure a signal generated in the brain or deliver stimulation to the brain, a transmission and reception portion configured to transmit, to the outside, a signal received from the contact portion or deliver, to the contact portion, a signal indicating the stimulation, and a connection portion connecting the contact portion and the transmission and reception portion; and an integrated circuit configured to exchange information delivered from the implantable element with the outside and receive power from the outside. The implantable element includes a brain wave collection device configured to collect brain wave information.

Specifically, the contact portion may form at least one electrode, and the brain wave collection device may include a converter (ADC) configured to convert analog information collected from the contact portion into digital information, a chip controller configured to control the converter, and a recorder configured to record information converted by the converter.

Specifically, the connection portion may include only two lines, a power line and a data line.

Specifically, in the connection portion, the power line may connect the chip controller and the integrated circuit, and the data line may connect the recorder and the integrated circuit.

Specifically, when waterproof-coating the contact portion, the system may waterproof-coat the converter, the chip controller, and the recorder together.

Specifically, the implantable element may be formed inside the skull, and the implantable element may be connected, through the connection portion, to the integrated circuit formed outside the skull or inserted into the skull.

Specifically, the integrated circuit may include a wireless power device and a wireless communication device. The wireless power device may be connected to the chip controller through the power line, and the wireless communication device may be connected to the recorder through the data line.

Specifically, the wireless power device may include a coil-shaped on-chip coil configured to wirelessly receive power from the outside, a current converter configured to convert alternating current transmitted from the on-chip coil into direct current, and a power management circuit configured to receive power from the current converter and control transmission of the power to the chip controller. The wireless communication device may include a wireless chip configured to wirelessly transmit and receive data.

Although the present invention has been described in detail through specific embodiments, the embodiments are provided for specifically describing the present invention, and the present invention is not limited thereto. It will be apparent that modifications or improvements can be made by a person of ordinary skill in the art within the technical spirit of the present invention.

Simple modifications or changes of the present invention all fall within the scope of the present invention, and the specific protection scope of the present invention will be clarified by the appended claims.

## Claims

1. A system including a cortical electrode and an inductive-coupling-based wireless device, the system comprising:
an implantable element including a contact portion configured to measure a signal generated in the brain or deliver stimulation to the brain, a transmission and reception portion configured to transmit, to the outside, a signal received from the contact portion or deliver, to the contact portion, a signal indicating the stimulation, and a connection portion connecting the contact portion and the transmission and reception portion; and
an integrated circuit module connected to the transmission and reception portion and configured to transmit and receive signals,
wherein the integrated circuit module has a stacked structure.

2. The system of claim 1, wherein the integrated circuit module comprises:
a housing; and
a stacked portion having the stacked structure,
wherein the housing comprises:
an upper case configured to protect an upper side of the stacked portion;
a side case having a height greater than a height of the stacked portion and including a through portion in at least a portion of one side thereof; and
a lower case formed below the stacked portion, and
wherein the side case is formed to be larger than the stacked portion such that a space is at least partially formed between the side case and the stacked portion.

3. The system of claim 2, wherein the transmission and reception portion is formed such that at least a portion thereof passes through the through portion.

4. The system of claim 2, wherein the upper case is formed of a synthetic resin material, and
wherein the side case and the lower case are formed of a metal material.

5. The system of claim 1, wherein the stacked portion comprises a receiver coil, a battery, an IC chip, a printed circuit board (PCB), and at least a portion of the transmission and reception portion.

6. The system of claim 5, wherein the transmission and reception portion included in the stacked portion is formed at a lowermost layer, and
wherein the stacked portion is stacked in the order of the transmission and reception portion, the PCB, the IC chip, the battery, and the receiver coil.

7. The system of claim 2, wherein the battery is formed in a circular shape, and
wherein the housing is provided in a rectangular shape.

8. The system of claim 6, wherein each component of the stacked portion is electrically connected.
